(19) Europäisches Patentamt
European Patent Office
Office européen des brevets

(11) Veröffentlichungsnummer: **0 593 541 B1**

# (12) EUROPÄISCHE PATENTSCHRIFT

(45) Veröffentlichungstag der Patentschrift: **20.09.95**

(51) Int. Cl.⁶: **C07C 255/30**, C08K 5/18, A61K 7/42, C07D 239/26, C07D 213/38

(21) Anmeldenummer: **92913649.7**

(22) Anmeldetag: **25.06.92**

(86) Internationale Anmeldenummer:
**PCT/EP92/01432**

(87) Internationale Veröffentlichungsnummer:
**WO 93/01164 (21.01.93 93/03)**

## (54) AMINOMETHYLENCYANESSIGSÄUREESTER UND -AMIDE.

(30) Priorität: **06.07.91 DE 4122475**

(43) Veröffentlichungstag der Anmeldung:
**27.04.94 Patentblatt 94/17**

(45) Bekanntmachung des Hinweises auf die Patenterteilung:
**20.09.95 Patentblatt 95/38**

(84) Benannte Vertragsstaaten:
**AT BE CH DE DK ES FR GB IT LI NL SE**

(56) Entgegenhaltungen:
**DE-A- 3 825 382**
**US-A- 3 079 366**

(73) Patentinhaber: **BASF Aktiengesellschaft**
**Carl-Bosch-Strasse 38**
**D-67063 Ludwigshafen (DE)**

(72) Erfinder: **HOLDERBAUM, Martin**
**Maudacher Strasse 366**
**D-6700 Ludwigshafen (DE)**
Erfinder: **AUMUELLER, Alexander**
**Rieslingweg 25**
**D-6730 Neustadt (DE)**
Erfinder: **TRAUTH, Hubert**
**Milanstrasse 6**
**D-6724 Dudenhofen (DE)**

EP 0 593 541 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Vorliegende Erfindung betrifft neue Aminomethylencyanessigsäureester und -amide der allgemeinen Formel I

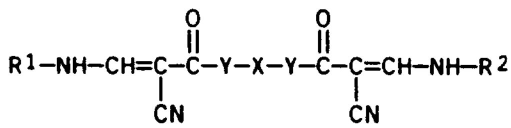

(I)

in der

- $R^1$ und $R^2$ unabhängig voneinander Phenyl, Naphthyl, Biphenyl oder fünf-oder sechsgliedriges Heteroaryl mit einem, zwei oder drei Stickstoffatomen oder einem Sauerstoffatom oder einem Schwefelatom oder einem Stickstoff- und einem Sauerstoffatom oder einem Stickstoff- und einem Schwefelatom, das benzanelliert sein kann, bedeuten, wobei diese Reste durch ein bis drei $C_1$- bis $C_{12}$-Alkylgruppen, $C_1$- bis $C_{12}$-Alkoxygruppen, Halogenatome, Cyanogruppen, Hydroxylgruppen oder Gruppen der Formeln $COOR^3$, $COR^3$, $CONHR^3$, $OCOR^3$ oder $NHCOR^3$ substituiert sein können und wobei
  $R^3$ für $C_1$- bis $C_{12}$-Alkyl, $C_5$- bis $C_8$-Cycloalkyl oder Phenyl steht,
- X eine $C_2$- bis $C_{30}$-Alkylengruppe, welche durch nicht benachbarte Sauerstoffatome unterbrochen sein kann, eine $C_4$- bis $C_{12}$-Alkenylen- oder $C_4$- bis $C_{12}$-Alkinylengruppe, wobei die ungesättigten Bindungen nicht zu den Ester-Sauerstoffatomen benachbart sind, eine $C_5$- bis $C_8$-Cycloalkylengruppe oder eine Phenylengruppe bezeichnet und
- Y für O oder NH steht.

Weiterhin betrifft die Erfindung ein verfahren zur Herstellung der Verbindungen I sowie die Verbindungen I enthaltende und somit gegen die Einwirkung von Licht, Sauerstoff und Wärme stabilisierte organische Materialien, insbesondere stabilisierte Kunststoffe und Lacke und die Verbindungen I als Lichtschutzmittel enthaltende kosmetische Zubereitungen.

Organisches Material, insbesondere Kunststoffe und Lacke, wird bekanntermaßen sehr schnell, vor allem durch Einwirkung von Licht, zerstört. Diese Zerstörung zeigt sich üblicherweise in Vergilbung, Verfärbung, Rißbildung oder Versprödung des Materials. Mit den bisher verwendeten Lichtschutzmitteln und Stabilisatoren konnte kein zufriedenstellender Schutz gegen die Zerstörung von organischem Material durch Licht, Sauerstoff und Wärme erzielt werden.

So werden beispielsweise in der US-A 3 079 366 u.a. Arylaminoethylene der Formel III

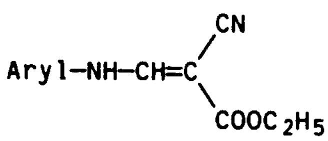

III

als UV-Absorber für Kunststoffe empfohlen. Die Verbindungen III weisen zwar die gewünschten spektroskopischen Eigenschaften auf, genügen jedoch hinsichtlich ihrer Stabilisierungs- bzw. Lichtschutzwirkung nicht den heute gestellten Anforderungen. Insbesondere ist die Vergilbungsneigung der mit den Verbindungen III stabilisierten Kunststoffe immer noch zu hoch.

Aufgabe der vorliegenden Erfindung war es daher, Lichtschutzmittel bzw. Stabilisatoren bereitzustellen, die einen wirkungsvollen Schutz für organisches Material mit sich bringen.

Demgemäß wurde die eingangs definierten Aminomethylencyanessigsäureester und -amide I gefunden.

Als Heteroarylreste $R^1$ bzw. $R^2$ kommen beispielsweise in Betracht: Pyrrol, Pyrazol, Imidazol, 1,2,3-Triazol, 1,2,4-Triazol, Pyridin, Pyridazin, Pyrimidin, Pyrazin, 1,2,3-Triazin, 1,2,4-Triazin, 1,3,5-Triazin, Furan, Thiophen, Oxazol, Isoxazol, Thiazol, Indol, Benzofuran, Benzothiophen, Benzimidazol, Chinolin, Isochinolin, Cinnolin, Chinazolin, Chinoxalin oder Phthalazin.

Als Substituenten an den Aryl- oder Heteroarylresten $R^1$ bzw. $R^2$ eignen sich:

- geradkettige oder verzweigte $C_1$- bis $C_{12}$-Alkylgruppen wie vor allem Methyl, Ethyl, n-Propyl, iso-Propyl, n-Butyl, isoButyl, sec.-Butyl und tert.-Butyl, daneben aber auch n-Pentyl, tert.-Pentyl, n-Hexyl, n-Heptyl, n-Octyl, 2-Ethylhexyl, n-Nonyl, iso-Nonyl, n-Decyl, iso-Decyl, n-Undecyl oder n-Dodecyl;

- geradkettige oder verzweigte $C_1$- bis $C_{12}$-Alkoxygruppen wie vor allem Methoxy, Ethoxy, n-Propoxy, iso-Propoxy, n-Butoxy, iso-Butoxy, sec.-Butoxy, n-Pentoxy und tert.-Pentoxy, daneben aber auch n-Hexoxy, n-Heptoxy, n-Octoxy, 2-Ethylhexoxy, n-Nonoxy, iso-Nonoxy, n-Decyloxy, iso-Decyloxy, n-Undecyloxy oder n-Dodecyloxy;
- Halogenatome wie vor allem Chlor, daneben aber auch Fluor, Brom oder Jod;
- Cyanogruppen;
- Hydroxylgruppen;
- Carbonestergruppen der Formel $COOR^3$, beispielsweise Methoxycarbonyl, Ethoxycarbonyl, Propoxycarbonyl oder Butoxycarbonyl;
- Acylgruppen der Formel $COR^3$, beispielsweise Acetyl oder Benzoyl;
- Carbonamidgruppen der Formel $CONHR^3$, beispielsweise N-Methylaminocarbonyl, N-Ethylaminocarbonyl oder N-Phenylaminocarbonyl;
- Carbonyloxygruppen der Formel $OCOR^3$, beispielsweise Methoxycarbonyloxy oder Ethoxycarbonyloxy;
- Carbonylaminogruppen der Formel $NHCOR^3$, beispielsweise N-Acetylamino, N-Propionylamino oder N-Benzoylamino.

Die Reste $R^3$ bedeuten hierbei geradkettige oder verzweigte $C_1$- bis $C_{12}$-Alkylgruppen, wofür die gleichen Beispiele wie oben angeführt werden können, $C_5$- bis $C_8$-Cycloalkyl wie vor allem Cyclopentyl und Cyclohexyl, daneben aber auch Cycloheptyl, Cyclooctyl, Methylcyclopentyl, Dimethylcyclopentyl, Methylcyclohexyl oder Dimethylcyclohexyl, sowie Phenyl.

Die Anzahl der Substituenten an den Aryl- oder Heteroarylresten $R^1$ bzw. $R^2$ kann bis zu 3, vorzugsweise bis zu 2 betragen. Bei mehreren Substituenten können diese gleich oder verschieden sein.

In einer bevorzugten Ausführungsform bedeuten die Reste $R^1$ und $R^2$ unabhängig voneinander vor allem Phenyl, welches durch ein oder zwei $C_1$- bis $C_{12}$-Alkylgruppen, insbesondere $C_1$- bis $C_4$-Alkylgruppen, $C_1$- bis $C_{12}$-Alkoxygruppen, insbesondere $C_1$- bis $C_5$-Alkoxygruppen, Chloratome, Cyanogruppen, Hydroxylgruppen oder Gruppen der Formel $COOR^3$, wobei $R^3$ die obengenannten Bedeutungen hat, insbesondere aber für $C_1$- bis $C_4$-Alkyl, Cyclopentyl, Cyclohexyl oder Phenyl steht, substituiert sein kann, daneben aber auch Pyridinyl- oder 2-Pyrimidinyl-Reste, welche durch ein oder zwei $C_1$-$C_4$-Alkylgruppen, insbesondere Methyl oder Ethyl, substituiert sein können.

Als Brückenglieder X eignen sich:

- geradkettige oder verzweigte $C_2$- bis $C_{30}$-Alkylengruppen wie 1,2-Ethylen, 1,2-Propylen, 1,3-Propylen, 1,4-Butylen, 1,2-Butylen, 2,3-Butylen, Pentamethylen, Hexamethylen, Heptamethylen, Octamethylen, Nonamethylen, Decamethylen, Undecamethylen oder Dodecamethylen;
- durch ein oder bis zu 9, insbesondere ein oder bis zu 5 nicht benachbarte Sauerstoffatome unterbrochene, vorzugsweise geradkettige oder nur in geringem Maße verzweigte $C_2$- bis $C_{30}$-Alkylengruppen, insbesondere $C_2$- bis $C_{18}$-Alkylengruppen;
- $C_4$- bis $C_{12}$-Alkenylengruppen wie 1,4-But-2-enylen, 1,6-Hex-3-enylen oder 1,8-Oct-4-enylen;
- $C_4$- bis $C_{12}$-Alkinylengruppen wie 1,4-But-2-inylen, 1,6-Hex-3-inylen oder 1,8-Oct-4-inylen;
- $C_5$- bis $C_8$-Cycloalkylengruppen wie 1,3-Cyclopentylen, 1,2-Cyclohexylen, 1,3-Cyclohexylen, 1,4-Cyclohexylen, 1,4-Cycloheptylen, 1,5-Cyclooctylen oder Gruppierungen der Formeln

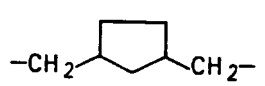 , 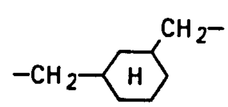 oder 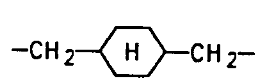 ;

- o-, m- oder vor allem p-Phenylen.

In einer bevorzugten Ausführungsform bezeichnen die Brückenglieder X

- eine $C_2$- bis $C_{12}$-Alkylengruppe, insbesondere eine $C_2$- bis $C_6$-Alkylengruppe, die vorzugsweise eine Polymethylengruppe ist;
- eine Polyethylenglykol- oder Polypropylenglykol-Gruppe der Formel $-(CH_2CH_2O)_n-CH_2CH_2-$, bzw.

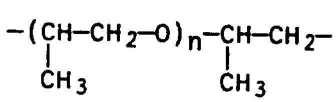

wobei n für 1 bis 9, insbesondere 1 bis 5, steht (die Werte für n stellen in der Regel Durchschnittswerte dar);

- eine cis-1,4-But-2-enylengruppe;
- eine 1,4-But-2-inylengruppe;
- eine $C_6$- bis $C_8$-Cycloalkylengruppe, insbesondere eine 1,2-Cyclohexylen- oder 1,4-Cyclohexylengruppe oder eine Gruppierung der Formel

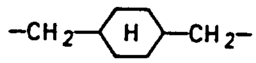 ;

- eine p-Phenylengruppe.

Die Herstellung der Verbindungen I erfolgt vorteilhafterweise durch Umsetzung von Cyanessigsäureestern oder -amiden der allgemeinen Formel II

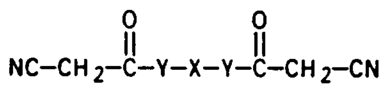 (II)

in der die Variablen X und Y die oben genannten Bedeutungen haben, mit 2 Äquivalenten aromatischer oder heteroaromatischer Amine der allgemeinen Formel $R^1$-$NH_2$ bzw. $R^2$-$NH_2$, in der die Reste $R^1$ und R2 die oben genannten Bedeutungen haben und mindestens 2 Äquivalenten eines Trialkylorthoformiates.

Als Trialkylorthoformiate haben sich Trimethylorthoformiat und vor allem Triethylorthoformiat bewährt.

Die Umsetzung wird zweckmäßigerweise in einem geeigneten polaren organischen Lösungsmittel wie einem Alkohol, z.B. n-Propanol, n-Butanol, Ethylenglykol, Diethylenglykol, Ethylenglykolmonomethylether, Cyclohexanol oder ähnlichen Verbindungen, vorgenommen. Als Lösungsmittel eignen sich auch Carbonsäureamide wie Dimethylformiat oder überschüssiges Trialkylorthoformiat. Bilden die verwendeten Ausgangsverbindungen bereits eine flüssige Mischung, kann auf ein zusätzliches Lösungsmittel verzichtet werden.

Man führt die Umsetzung in der Regel bei Temperaturen von 70 bis 180 °C, vorzugsweise 100 bis 150 °C, und bei Normaldruck durch. Die drei Reaktionspartner werden im angegebenen stöchiometrischen oder annähernd stöchiometrischen Verhältnis eingesetzt, wenn das Trialkylorthoformiat nicht zusätzlich als Lösungsmittel dient; leichte Überschüsse des einen oder anderen Reaktionspartners, etwa bis zu 15 %, können dabei in Kauf genommen werden.

Als Katalysatoren für die Umsetzung können bei sehr langen Reaktionszeiten gewünschtenfalls zusätzlich Lewis-Säuren wie $AlCl_3$, $ZrCl_4$, $TiCl_4$ oder vor allem $ZnCl_2$ in den hierfür üblichen Mengen verwendet werden.

Die Cyanessigsäuresäureester II können beispielsweise durch Umsetzung von Cyanessigsäure mit entsprechenden Diolen HO-X-OH in Gegenwart eines Katalysators wie Borsäure oder Tetrabutylorthotitanat hergestellt werden und sind z.B. aus der US-A 4 218 515 und der EP-A 136 260 bekannt.

Die erfindungsgemäßen Aminomethylencyanessigsäureester und -amide I eignen sich in hervorragender Weise zum Stabilisieren von organischem Material gegen die Einwirkung von Licht, Sauerstoff und Wärme. Sie sind auch wirksam als Metalldesaktivator. Sie werden dem zu stabilisierenden organischen Material in einer Konzentration von 0,01 bis 5 Gew.%, vorzugsweise von 0,02 bis 2 Gew.%, bezogen auf das organische Material, vor, während oder nach seiner Herstellung zugesetzt.

Unter organischem Material sind beispielsweise kosmetische Präparate wie Salben und Lotionen, Arzneimittelformulierungen wie Pillen und Zäpfchen oder Vorprodukte für Kunststoffe und Lacke, insbesondere jedoch Kunststoff und Lacke selbst, zu verstehen.

Gegenstand der vorliegenden Erfindung ist außerdem gegen die Einwirkung von Licht, Sauerstoff und Wärme stabilisiertes organisches Material, insbesondere Kunststoff und Lacke, welches die Verbindungen I in den oben angegebenen Konzentrationen enthält.

Zur Vermischung der erfindungsgemäßen Verbindungen I vor allem mit Kunststoffen können alle bekannten Vorrichtungen und Methoden zum Einmischen von Stabilisierungsmitteln oder anderen Zusätzen in Polymere angewandt werden.

Das durch die erfindungsgemäßen Verbindungen I stabilisierte organische Material kann gegebenenfalls noch weitere Additive enthalten, z.B. Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den erfindungsgemäßen Verbindungen zugesetzt werden können, sind z.B. Verbindungen auf der Basis sterisch gehinderter Phenole oder Schwefel oder

Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionylethyl]isocyanurat, 1,3,5-Tris-(2,6-dimethyl-3-hydroxy-4-tert.-butylbenzyl)-isocyanuratund Pentaerythrit-tetrakis-[β-(3,5-di-tert.-butyl-4-hydroxy-phenyl)-propionat] erwähnt.

Als phosphorhaltige Antioxidantien kommen beispielsweise Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat) genannt.

Weitere Antioxidantien und Lichtstabilisatoren, die zusammen mit den Verbindungen I verwendet werden können, sind z.B. 2-(2'-Hydroxyphenyl)-benztriazole, 2-Hydroxybenzophenone, Arylester von Hydroxybenzoesäuren, α-Cyanozimtsäurederivate, Benzimidazolcarbonsäureanilide, Nickelverbindungen oder Oxalsäuredianilide.

Eine besonders gute Stabilisierung erhält man, wenn zu den Verbindungen I noch mindestens einen Lichtstabilisator aus der Verbindungsklasse der sterisch gehinderten Amine in üblicher Konzentration zusetzt.

Als sterisch gehinderte Amine kommen z.B. in Betracht: Bis-(2,2,6,6-tetramethylpiperidyl)-sebacat, Bis-(1,2,2,6,6-pentamethylpiperidyl)-sebacat, Bis-(1,2,2,6,6-pentamethylpiperidyl)-ester, das Kondensationsprodukt von 1-Hydroxyethyl-2,2,6,6-tetramethyl-4-hydroxypiperidin und Bernsteinsäure, das Kondensationsprodukt von N,N'-(2,2,6,6-Tetramethylpiperidyl)-hexamethylendiaminund 4-tert.-Octylamino-2,6-dichlor-1,3,5-s-triazin, Tris-(2,2,6,6-Tetramethylpiperidyl)-nitrilotriacetat, Tetrakis-(2,2,6,6-tetramethyl-4-piperidyl)-1,2,3,4-butan-tetracarbonsäure, 1,1'-(1,2-Ethandiyl)-bis-(3,3,5,5-tetramethylpiperazinon), die Kondensationsprodukte von 4-Amino-2,2,6,6-tetramethylpiperidinen und Tetramethylolacetylendiharnstoffen.

Als Kunststoffe, die durch die erfindungsgemäßen Verbindungen I stabilisiert werden können, seien beispielsweise genannt:

Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;

Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS);

halogenhaltige Polymere, wie z.B. Polyvinylchlorid, Polyvinylfluorid, Polyvinylidenfluorid sowie deren Copolymere;

Polymere, die sich von α,β-ungesättigten Säuren und deren Derivaten ableiten, wie Polyacrylate, Polymethacrylate, Polyacrylamide und Polyacrylnitrile;

Polymere, die sich von ungesättigten Alkoholen und Aminen bzw. von deren Acrylderivaten oder Acetalen ableiten, z.B. Polyvinylalkohol und Polyvinylacetat;

Polyurethane, Polyamide, Polyharnstoffe, Polyester, Polycarbonate, Polysulfone, Polyethersulfone und Polyetherketone.

Weiterhin können mit den erfindungsgemäßen Verbindungen I Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die Verbindungen I können in fester oder gelöster Form dem Lack zugesetzt werden. Ihre gute Löslichkeit in Lacksystemen ist dabei von besonderem Vorteil.

Auch bei der Verwendung als Stabilisatoren in Lacken können die bereits aufgeführten zusätzlichen Additive, insbesondere Antioxidantien und Lichtstabilisatoren, mitverwendet werden.

Die erfindungsgemäßen Verbindungen I eignen sich in besonderem Maße zur Stabilisierung von Polystyrol, Copolymeren aus Styrol-Acrylnitril (SAN) und Acrylnitril-Butadien-Styrol (ABS), Polyurethanen, Polyamiden, Polyestern, Polyolefinen sowie von Lacken.

Eine ganz besonders gute Stabilisierung von Polyurethanen erhält man, wenn das Polyurethan mit einem Gemisch aus mindestens einer Verbindung I, mindestens einem der oben genannten Antioxidantien und mindestens einer der oben genannten sterisch gehinderten Aminverbindungen stabilisiert wird.

Weiterhin eignen sich die erfindungsgemäßen Aminomethylencyanessigsäureester und -amide I auch als Lichtschutzmittel in kosmetischen Zubereitungen, also insbesondere zum vorsorglichen Schutz der menschlichen Haut vor der schädigenden Einwirkung von Licht, speziell Sonnenlicht, aber auch künstlichem Licht, welches hohe UV-Anteile aufweist. Unter organischen Materialien ist im weitesten Sinne somit auch die menschliche Haut zu verstehen. Die kosmetischen Zubereitungen als solche werden zugleich natürlich auch stabilisiert, um möglichst lange wirksam zu bleiben.

Demgemäß sind auch Gegenstand der vorliegenden Erfindung kosmetische Zubereitungen, welche 0,1 bis 10 Gew.%, vorzugsweise 1 bis 7 Gew.%, bezogen auf die Menge der kosmetischen Zubereitung, eines oder mehrerer Aminomethylencyanessigsäureester und -amide I als Lichtschutzmittel enthalten. Derartige kosmetische Zubereitungen sind beispielsweise Sonnenschutzpräparate in flüssiger, fester oder pastöser Form wie Cremes, Lotionen, Aerosol-Schaumcremes, Gele, Öle, Fettstifte, Puder oder Sprays.

Die Verbindungen I werden in den kosmetischen Zubereitungen in den üblichen Trägermedien oder Verdünnungsmitteln eingesetzt, beispielsweise als Lösung in einem kosmetischen Öl. Übliche Ölkomponenten in der Kosmetik sind beispielsweise Paraffinöl, Glycerylstearat, Isopropylmyristat, Diisopropyladipat, 2-Ethylhexansäure-cetylstearylester, hydriertes Polyisobuten, Vaseline, Caprylsäure/Caprinsäure-Triglyceride, mikrokristallines Wachs, Lanolin und Stearinsäure. Von besonderem Vorteil ist die gute Löslichkeit der Verbindungen I in diesen Ölkomponenten.

Die erfindungsgemäßen Verbindungen I zeichnen sich durch eine gute Verträglichkeit mit den üblichen Kunststoffarten und durch eine gute Löslichkeit in den üblichen Lacksystemen und in den üblichen kosmetischen Ölen aus. Sie haben in der Regel keine oder nur eine sehr geringe Eigenfarbe, sind bei den üblichen Kunststoff- und Lack-Verarbeitungstemperaturen stabil und nicht flüchtig, zeigen eine nur geringe Migrationsneigung und bewirken vor allen Dingen eine lange Schutzdauer der mit ihnen behandelten organischen Materialien.

Herstellungsbeispiele

Beispiel 1

21,0 g (0,10 mol) 1,3-Propandioldicyanoacetat, 19,6 g (0,21 mol) Anilin sowie 34,1 g (0,23 mol) Triethylorthoformiat wurden in 50 ml Ethylenglykol 2 h auf 110 °C erhitzt. Es wurden langsam 38 ml Ethanol abdestilliert, wobei die Temperatur auf 140 °C anstieg. Danach wurde auf 80 °C abgekühlt, 100 ml Methanol wurden zugesetzt und es wurde auf Raumtemperatur abgekühlt. Der Niederschlag wurde abfiltriert und mit Methanol gewaschen. Zur weiteren Reinigung wurde mit Methanol ausgekocht. Man erhielt 27,3 g Produkt (entsprechend einer Ausbeute von 66 %) vom Schmelzpunkt 164-66 °C.

Die spektroskopischen Daten sind in Tabelle 1 angegeben.

Beispiele 2 bis 57

Analog zu Beispiel 1 wurden unter Verwendung der entsprechenden aromatischen oder heteroaromatischen Amine $R^1$-$NH_2$ bzw. $R^2$-$NH_2$ die in Tabelle 1 aufgeführten Produkte aus entsprechenden Cyanessigsäureestern II und Triethylorthoformiat hergestellt. Die Schmelzpunkte und die spektroskopischen Daten der Produkte sind ebenfalls in Tabelle 1 angegeben.

Tabelle 1

Struktur, Schmelzpunkt und spektroskopische Daten der hergestellten Aminomethylencyanessigsäureester I

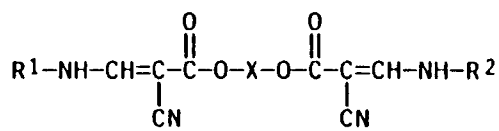

(I)

| Bsp. Nr. | Struktur $R^1$ | $R^2$ | X | Schmelzpunkt [°C] | UV-Daten ($CH_2Cl_2$) $\lambda_{max}$ [nm] | ε |
|---|---|---|---|---|---|---|
| $R^1$, $R^2$ = durch folgende Gruppen substituiertes Phenyl: | | | | | | |
| 1 | H | H | $(CH_2)_3$ | 164-66 | 321 | 53800 |
| 2 | $4-COOC_2H_5$ | $4-COOC_2H_5$ | $(CH_2)_3$ | 228-30 | 333 | 79800 |
| 3 | $3-CH_3$ $5-CH_3$ | $3-CH_3$ $5-CH_3$ | $(CH_2)_3$ | 246-48 | 324 | 34200 |
| 4 | $2-CH_3$ $4-CH_3$ | $2-CH_3$ $4-CH_3$ | $(CH_2)_3$ | 185 | 330 | 46900 |
| 5 | $4-CH_3$ | $4-CH_3$ | $(CH_2)_3$ | 186 | 326 | 51400 |
| 6 | $4-OCH_3$ | $4-OCH_3$ | $(CH_2)_3$ | 189 | 333 | 45400 |
| 7 | $4-OCH_3$ | $2-CH_3$ $4-OCH_3$ | $(CH_2)_3$ | 175 | 335 | 41400 |
| 8 | H | H | $(CH_2)_2$ | 219 | 323 | 51700 |

Tabelle 1 (Forts.)

| Bsp. Nr. | Struktur $R^1$ | $R^2$ | X | Schmelzpunkt [°C] | UV-Daten ($CH_2Cl_2$) $\lambda_{max}$ [nm] | ε |
|---|---|---|---|---|---|---|
| 9 | $4-COOC_2H_5$ | $4-COOC_2H_5$ | $(CH_2)_2$ | 246-48 | 330 | |
| 10 | $3-CH_3$ $5-CH_3$ | $3-CH_3$ $5-CH_3$ | $(CH_2)_2$ | 225-28 | 325 | 49500 |
| 11 | $2-CH_3$ $4-CH_3$ | $2-CH_3$ $4-CH_3$ | $(CH_2)_2$ | 222 | 331 | 44300 |
| 12 | $4-CH_3$ | $4-CH_3$ | $(CH_2)_2$ | 260 | 327 | 51900 |
| 13 | $4-OCH_3$ | $4-OCH_3$ | $(CH_2)_2$ | 262 | 332 | |
| 14 | $2-CH_3$ $4-OCH_3$ | $2-CH_3$ $4-OCH_3$ | $(CH_2)_2$ | 220 | 335 | 37800 |
| 15 | H | H | $(CH_2)_4$ | 189 | 321 | 51200 |
| 16 | $4-COOC_2H_5$ | $4-COOC_2H_5$ | $(CH_2)_4$ | 246 | 334 | 64600 |
| 17 | $4-CH_3$ | $4-CH_3$ | $(CH_2)_4$ | 212 | 327 | 52200 |
| 18 | $2-CH_3$ $4-CH_3$ | $2-CH_3$ $4-CH_3$ | $(CH_2)_4$ | 226 | 331 | 46500 |
| 19 | $4-OCH_3$ | $4-OCH_3$ | $(CH_2)_4$ | 219 | 332 | 45300 |
| 20 | $3-CH_3$ $5-CH_3$ | $3-CH_3$ $5-CH_3$ | $(CH_2)_4$ | 239 | 324 | 52500 |
| 21 | H | H | $(CH_2)_6$ | 145 | 321 | 54900 |

Tabelle 1 (Forts.)

| Bsp. Nr. | Struktur $R^1$ | $R^2$ | X | Schmelzpunkt [°C] | UV-Daten ($CH_2Cl_2$) $\lambda_{max}$ [nm] | ε |
|---|---|---|---|---|---|---|
| 22 | $4-COOC_2H_5$ | $4-COOC_2H_5$ | $(CH_2)_6$ | 232 | 333 | 77700 |
| 23 | $3-CH_3$<br>$5-CH_3$ | $3-CH_3$<br>$5-CH_3$ | $(CH_2)_6$ | 188 | 324 | 53000 |
| 24 | $2-CH_3$<br>$4-CH_3$ | $2-CH_3$<br>$4-CH_3$ | $(CH_2)_6$ | 223 | 330 | 44900 |
| 25 | $4-CH_3$ | $4-CH_3$ | $(CH_2)_6$ | 193 | 323 | 50700 |
| 26 | $4-OCH_3$ | $4-OCH_3$ | $(CH_2)_6$ | 163 | 331 | 45800 |
| 27 | 4-Cl | 4-Cl | $(CH_2)_6$ | 191 | 324 | 46200 |
| 28 | 4-CN | 4-CN | $(CH_2)_6$ | 268-71 | 330 | |
| 29 | H | H | 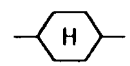 | 255-64 | 324 | 62700 |
| 30 | $4-OCH_3$ | $4-OCH_3$ | 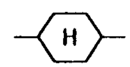 | 265 | 333 | 44800 |
| 31 | $4-COOC_2H_5$ | $4-COOC_2H_5$ | 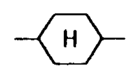 | 239-47 | 336 | 81700 |

Tabelle 1 (Forts.)

| Bsp. Nr. | Struktur $R^1$ | $R^2$ | X | Schmelzpunkt [°C] | UV-Daten ($CH_2Cl_2$) $\lambda_{max}$ [nm] | ε |
|---|---|---|---|---|---|---|
| 32 | 3-$CH_3$ 5-$CH_3$ | 3-$CH_3$ 5-$CH_3$ | 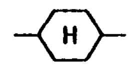 | 259-64 | 325 | 51200 |
| 33 | 2-$CH_3$ 4-$CH_3$ | 2-$CH_3$ 4-$CH_3$ | 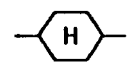 | 247 | 332 | 48400 |
| 34 | 4-$CH_3$ | 4-$CH_3$ | 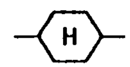 | 280 | 326 | 53500 |
| 35 | 4-CN | 4-CN | 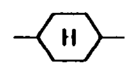 | 310 | 334 | 61700 |
| 36 | H | H | 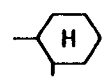 | 113 | 320 | 49700 |
| 37 | H | H | $CH_2$-CH=CH-$CH_2$ | 139 | 322 | 54400 |
| 38 | 4-$COOC_2H_5$ | 4-$COOC_2H_5$ | $CH_2$-CH=CH-$CH_2$ | 183 | 332 | 77400 |

Tabelle 1 (Forts.)

| Bsp. Nr. | Struktur $R^1$ | $R^2$ | X | Schmelzpunkt [°C] | UV-Daten ($CH_2Cl_2$) $\lambda_{max}$ [nm] | ε |
|---|---|---|---|---|---|---|
| 39 | $3-CH_3$ $5-CH_3$ | $3-CH_3$ $5-CH_3$ | $CH_2-CH=CH-CH_2$ | 137 | 325 | 52400 |
| 40 | $2-CH_3$ $4-CH_3$ | $2-CH_3$ $4-CH_3$ | $CH_2-CH=CH-CH_2$ | 206 | 330 | 48600 |
| 41 | $4-CH_3$ | $4-CH_3$ | $CH_2-CH=CH-CH_2$ | 184 | 326 | 52700 |
| 42 | 4-Cl | 4-Cl | $CH_2-CH=CH-CH_2$ | 213 | 326 | 59700 |
| 43 | H | H | $CH_2-C\equiv C-CH_2$ | 185 | 322 | 52400 |
| 44 | $4-COOC_2H_5$ | $4-COOC_2H_5$ | $CH_2-C\equiv C-CH_2$ | 242 | 332 | 79100 |
| 45 | $3-CH_3$ $5-CH_3$ | $3-CH_3$ $5-CH_3$ | $CH_2-C\equiv C-CH_2$ | 239 | 324 | |
| 46 | $2-CH_3$ $4-CH_3$ | $2-CH_3$ $4-CH_3$ | $CH_2-C\equiv C-CH_2$ | 249 | 331 | 45000 |
| 47 | $4-CH_3$ | $4-CH_3$ | $CH_2-C\equiv C-CH_2$ | 244 | 327 | 50500 |
| 48 | 4-Cl | 4-Cl | $CH_2-C\equiv C-CH_2$ | 243 | 326 | 54600 |
| 49 | $2-CH_3$ 5-Cl | $2-CH_3$ 5-Cl | $(CH_2)_6$ | 205 | 328 | 48500 |
| 50 | H | H | $CH_2$– 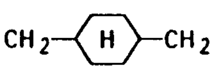 –$CH_2$ | 257 | 320 | |

Tabelle 1 (Forts.)

| Bsp. Nr. | Struktur $R^1$ | $R^2$ | X | Schmelzpunkt [°C] | UV-Daten ($CH_2Cl_2$) $\lambda_{max}$ [nm] | ε |
|---|---|---|---|---|---|---|
| 51 | $4-COOC_2H_5$ | $4-COOC_2H_5$ | $CH_2$– 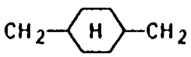 –$CH_2$ | 263 | 329 | |
| 52 | $2-OCH_3$ $4-OCH_3$ | $2-OCH_3$ $4-OCH_3$ | $(CH_2)_6$ | 242-45 | 348 | |
| 53 | H | H | $(CH_2CH_2O)_{4,2}-CH_2CH_2$ | (Harz) | 321 | |
| $R^1$ = $R^2$ = Heteroaryl: | | | | | | |
| 54 | 3-Pyridinyl | | $(CH_2)_2$ | 200 | 318 | |
| 55 | 4,6-Dimethyl-2-pyrimidinyl | | $(CH_2)_2$ | 228 | 316 | 63400 |
| 56 | 4,6-Dimethyl-2-pyrimidinyl | | 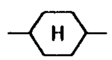 | 262-65 | 315 | 71700 |
| 57 | 4,6-Dimethyl-2-pyrimidinyl | | $(CH_2)_4$ | 203 | 314 | 64800 |

Anmerkung: Die Doppelbindung in der 1,4-But-2-enylengruppe für X bei den Beispielen Nr. 37 bis 42 ist cis-ständig.

Beispiele 58 bis 80

Analog zu Beispiel 1 wurden unter Verwendung der entsprechenden aromatischen oder heteroaromatischen Amine $R^1-NH_2$ bzw. $R^2-NH_2$ die in Tabelle 2 aufgeführten Produkte aus entsprechenden

Cyanessigsäurebisamiden II und Triethylorthoformiat hergestellt. Die Schmelzpunkte und die spektroskopischen Daten der Produkte sind ebenfalls in Tabelle 2 angegeben.

Tabelle 2

Struktur, Schmelzpunkt und spektroskopische Daten der hergestellten Aminomethylencyanessigsäureamide I

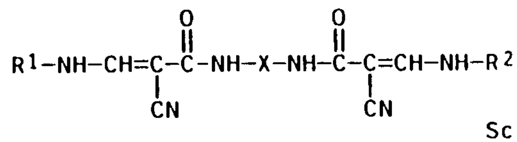

(I)

| Bsp. Nr. | Struktur $R^1 = R^2$ | X | Schmelzpunkt [°C] | UV-Daten ($CH_2Cl_2$) $\lambda_{max}$ [nm] | ε |
|---|---|---|---|---|---|
| 58 | Phenyl | $(CH_2)_6$ | 202 | 323 | 57500 |
| 59 | 3,5-Dimethylphenyl | $(CH_2)_6$ | 233 | 326 | 57800 |
| 60 | 4-Ethoxycarbonylphenyl | $(CH_2)_6$ | 205 | 334 | 75000 |
| 61 | 4-Methoxyphenyl | $(CH_2)_6$ | 215 | 332 | 48600 |
| 62 | 4-Methylphenyl | $(CH_2)_6$ | 210 | 326 | 54100 |
| 63 | 2,4-Dimethylphenyl | $(CH_2)_6$ | 198 | 330 | 51000 |
| 64 | Phenyl | 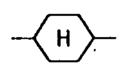 | 306 | 327 | 53300 |
| 65 | 4-Methylphenyl | 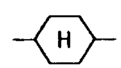 | 293 | 329 | 60100 |

Tabelle 2 (Forts.)

| Bsp. Nr. | Struktur $R^1 = R^2$ | X | Schmelzpunkt [°C] | UV-Daten ($CH_2Cl_2$) $\lambda_{max}$ [nm] | ε |
|---|---|---|---|---|---|
| 66 | 4-Methoxyphenyl | 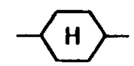 | 295 | 334 | 50500 |
| 67 | 3,5-Dimethylphenyl | 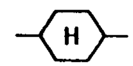 | 265 | 327 | 49400 |
| 68 | Phenyl | 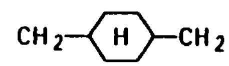 | 218 | 324 | 54000 |
| 69 | 4-Ethoxycarbonylphenyl | 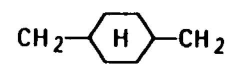 | 271 | 337 | 83600 |
| 70 | 4-Methoxyphenyl | 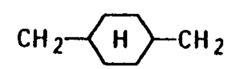 | 246 | 332 | 46300 |
| 71 | Phenyl | $(CH_2)_2$ | 268 | 325 | 56100 |
| 72 | 4-Ethoxycarbonylphenyl | $(CH_2)_2$ | 235 | 337 | 73200 |

Tabelle 2 (Forts.)

| Bsp. Nr. | Struktur R1 = R2 | X | Schmelzpunkt [°C] | UV-Daten ($CH_2Cl_2$) $\lambda_{max}$ [nm] | ε |
|---|---|---|---|---|---|
| 73 | 3,5-Dimethylphenyl | $(CH_2)_2$ | 261 | 327 | 56300 |
| 74 | 4-Methylphenyl | $(CH_2)_2$ | 263 | 328 | 57200 |
| 75 | 4-Methoxyphenyl | $(CH_2)_2$ | 244 | 333 | - |
| 76 | 4,6-Dimethyl-2-pyrimidinyl | $(CH_2)_2$ | 263 | 315 | 71500 |
| 77 | 4,6-Dimethyl-2-pyrimidinyl | 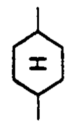 | 311 | 315 | - |
| 78 | Phenyl | $(CH_2)_3$ | 203 | 324 | 58400 |
| 79 | 4-Methylphenyl | $(CH_2)_3$ | 231 | 328 | 58600 |
| 80 | 4-Methoxyphenyl | $(CH_2)_3$ | 191 | 333 | 51000 |

Anwendungsbeispiele

Zur Herstellung von Belichtungsproben aus ABS wurden 0,5 Gew.-% des sterisch gehinderten Amins der Formel IV und 0,5 Gew.-% des in Tabelle 3 angegebenen erfindungsgemäßen UV-Absorbers in ABS vom Typ Terluran 967 K (umpigmentiert) durch einmaliges Extrudieren bei einer Massetemperatur von 250 °C gelöst und das anfallende Granulat wurde bei 260 °C zu 2 mm dicken Formkörpern gespritzt.

Die Spritzlinge wurden in einem Schnellbewitterungsgerät vom Typ Xenotest ® 1200 auf ihre Licht- und Wetterechtheit getestet.

Ein Maß für die Vergilbung und somit für den photooxidativen Abbau des Polymeren ist, in Abhängigkeit von der Bewitterungszeit, der Yellowness Index (YI), gemäß Annual Book of ASTM Standards D 1925-70 (Reapproved 1977), der mit zunehmender Vergilbung ansteigt.

Die verwendeten Stabilisatorengemische sowie die Ergebnisse der Belichtung sind in Tabelle 3 zusammengestellt.

Tabelle 3

YI-Werte von ABS-Proben

| Stabilisatorengemisch | YI-Wert Belichtung nach 500 h |
|---|---|
| erfindungsgemäß: | |
| 0,5 Gew.-% IV + 0,5 Gew.-% Produkt aus Bsp. 6 | 28 |
| 0,5 Gew.-% IV + 0,5 Gew.-% Produkt aus Bsp. 15 | 27 |
| 0,5 Gew.-% IV + 0,5 Gew.-% Produkt aus Bsp. 17 | 28 |
| zum Vergleich: | |
| ohne Stabilisatoren | 52 |
| 0,5 Gew.-% IV + 0,5 Gew.-% IIIa | 34 |

IIIa: 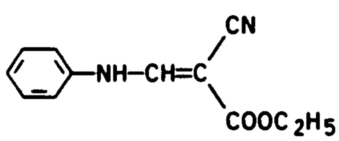 gemäß US-A 3 079 366

IV: 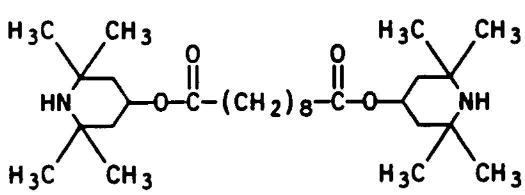

## Patentansprüche

**1.** Aminomethylencyanessigsäureester und -amide der allgemeinen Formel I

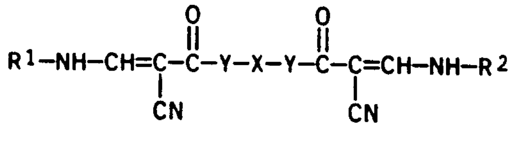 (I)

in der

$R^1$ und $R^2$ unabhängig voneinander Phenyl, Naphthyl, Biphenyl oder fünf-oder sechsgliedriges Heteroaryl mit einem, zwei oder drei Stickstoffatomen oder einem Sauerstoffatom oder einem Schwefelatom oder einem Stickstoff- und einem Sauerstoffatom oder einem Stickstoff- und einem Schwefelatom, das benzanelliert sein kann, bedeuten, wobei diese Reste durch ein bis drei $C_1$ -bis $C_{12}$-Alkylgruppen, $C_1$- bis $C_{12}$-Alkoxygruppen, Halogenatome, Cyanogruppen, Hydroxylgruppen oder Gruppen der Formeln $COOR^3$, $COR^3$, $CONHR^3$, $OCOR^3$ oder $NHCOR^3$ substituiert sein können und wobei $R^3$ für $C_1$- bis $C_{12}$-Alkyl, $C_5$- bis $C_8$-Cycloalkyl oder Phenyl steht,

X eine $C_2$- bis $C_{30}$-Alkylengruppe, welche durch nicht benachbarte Sauerstoffatome unterbrochen sein kann, eine $C_4$- bis $C_{12}$-Alkenylen- oder $C_4$- bis $C_{12}$-Alkinylengruppe, wobei die ungesättigten Bindungen nicht zu den Ester-Sauerstoffatomen benachbart sind, eine $C_5$- bis $C_8$-Cycloalkylengruppe oder eine Phenylengruppe bezeichnet und

Y für O oder NH steht.

**2.** Aminomethylencyanessigsäureester und -amide I nach Anspruch 1, bei denen $R^1$ und $R^2$ unabhängig voneinander Phenyl, welches durch ein oder zwei $C_1$- bis $C_{12}$-Alkylgruppen, $C_1$-bis $C_{12}$-Alkoxygruppen, Chloratome, Cyanogruppen, Hydroxylgruppen oder Gruppen der Formel $COOR^3$, wobei $R^3$ die obengenannten Bedeutungen hat, substituiert sein kann, oder Pyridinyl- oder 2-Pyrimidinyl-Reste, welche durch ein oder zwei $C_1$-bis $C_4$-Alkylgruppen substituiert sein können, bedeuten.

**3.** Aminomethylencyanessigsäureester und -amide I nach Anspruch 1 oder 2, bei denen X eine $C_2$- bis $C_{12}$-Alkylengruppe, eine Gruppe der Formel $-(CH_2CH_2O)_n-CH_2CH_2-$ oder

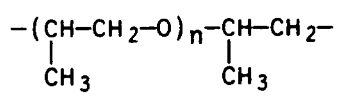

wobei n für 1 bis 9 steht, eine 1,4-But-2-enylengruppe, eine 1,4-But-2-inylengruppe, eine $C_6$- bis $C_8$-Cycloalkylengruppe oder eine Phenylengruppe bezeichnet.

**4.** Verfahren zur Herstellung von Aminomethylencyanessigsäureestern und -amiden I gemäß den Ansprüchen 1 bis 3, dadurch gekennzeichnet, daß man Cyanessigsäureester oder -amide der allgemeinen Formel II

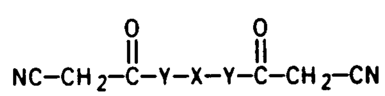

(II),

in der die Variablen X und Y die obengenannten Bedeutungen haben, mit 2 Äquivalenten aromatischer oder heteroaromatischer Amine der allgemeinen Formel $R^1-NH_2$ bzw. $R^2-NH_2$, in der die Reste $R^1$ und $R^2$ die obengenannten Bedeutungen haben, und mindestens 2 Äquivalenten eines Trialkylorthoformiates umsetzt.

**5.** Verwendung von Aminomethylencyanessigsäureestern und -amiden I gemäß den Ansprüchen 1 bis 3 als Stabilisatoren für organische Materialien.

**6.** Verwendung von Aminomethylencyanessigsäureestern und -amiden I gemäß den Ansprüchen 1 bis 3 als Stabilisatoren für Kunststoffe und Lacke.

**7.** Gegen die Einwirkung von Licht, Sauerstoff und Wärme stabilisierte organische Materialien, enthaltend 0,01 bis 5 Gew.-%, bezogen auf die Menge des organischen Materials, eines oder mehrerer Aminomethylencyanessigsäureester oder -amide I gemäß den Ansprüchen 1 bis 3.

**8.** Gegen die Einwirkung von Licht, Sauerstoff und Wärme stabilisierte Kunststoffe und Lacke, enthaltend 0,01 bis 5 Gew.-%, bezogen auf die Menge des Kunststoffes bzw. Lackes, eines oder mehrerer Aminomethylencyanessigsäureester oder -amide I gemäß den Ansprüchen 1 bis 3.

**9.** Kosmetische Zubereitungen, enthaltend 0,1 bis 10 Gew.-%, bezogen auf die Menge der kosmetischen Zubereitungen, eines oder mehrerer Aminomethylencyanessigsäureester oder -amide I gemäß den Ansprüchen 1 bis 3 als Lichtschutzmittel.

**10.** Verfahren zum Stabilisieren von organischen Materialien, dadurch gekennzeichnet, daß man hierzu Aminomethylencyanessigsäureester oder -amide I gemäß den Ansprüchen 1 bis 3 verwendet.

**11.** Verfahren zum Stabilisieren von Kunststoffen und Lacken, dadurch gekennzeichnet, daß man hierzu Aminomethylencyanessigsäureester oder -amide I gemäß den Ansprüchen 1 bis 3 verwendet.

**12.** Verfahren zum Schutz der menschlichen Haut vor der Einwirkung von Licht, dadurch gekennzeichnet, daß man hierzu kosmetische Zubereitungen verwendet, welche Aminomethylencyanessigsäureester oder -amide I gemäß den Ansprüchen 1 bis 3 als Lichtschutzmittel enthalten.

## Claims

**1.** An ester or amide of aminomethylenecyanoacetic acid of the formula I

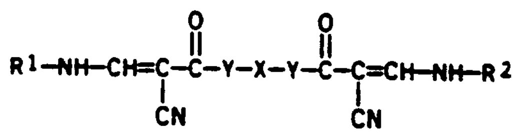

(I)

where

| | |
|---|---|
| $R^1$ and $R^2$ | are each, independently of one another, phenyl, naphthyl, biphenylyl or five- or six-membered hetaryl with one, two or three nitrogens or one oxygen or one sulfur or one nitrogen and one oxygen or one nitrogen and one sulfur, which can be benzo-fused, it being possible for these radicals to be substituted by one to three $C_1$-$C_{12}$-alkyl groups, $C_1$-$C_{12}$-alkoxy groups, halogen atoms, cyano groups, hydroxyl groups or groups of the formulae $COOR^3$, $COR^3$, $CONHR^3$, $OCOR^3$ or $NHCOR^3$, and where $R^3$ is $C_1$-$C_{12}$-alkyl, $C_5$-$C_8$-cycloalkyl or phenyl, |
| X | is $C_2$-$C_{30}$-alkylene which can be interrupted by non-adjacent oxygens, or is $C_4$-$C_{12}$-alkenylene or $C_4$-$C_{12}$-alkynylene where the unsaturated bonds are not adjacent to the ester oxygens, or is $C_5$-$C_8$-cycloalkylene or phenylene, and |
| Y | is O or NH. |

**2.** An ester or amide of aminomethylenecyanoacetic acid I as claimed in claim 1, where $R^1$ and $R^2$ are each, independently of one another, phenyl which can be substituted by one or two $C_1$-$C_{12}$-alkyl groups, $C_1$-$C_{12}$-alkoxy groups, chlorine atoms, cyano groups, hydroxyl groups or groups of the formula $COOR^3$ where $R^3$ has the abovementioned meanings, or pyridinyl or 2-pyrimidinyl, each of which can be substituted by one or two $C_1$-$C_4$-alkyl groups.

**3.** An ester or amide of aminomethylenecyanoacetic acid I as claimed in claim 1 or 2, where X is $C_2$-$C_{12}$-alkylene, $-(CH_2CH_2O)_n-CH_2CH_2-$ or

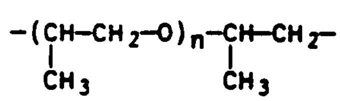

where n is 1 to 9, 1,4-but-2-enylene, 1,4-but-2-ynylene, $C_6$-$C_8$-cycloalkylene or phenylene.

**4.** A process for preparing esters or amides of aminomethylenecyanoacetic acid I as claimed in claims 1 to 3, which comprises reacting cyanoacetic esters or amides of the formula II

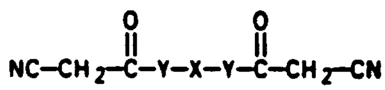

(II)

where X and Y have the abovementioned meanings,
with 2 equivalents of aromatic or heteroaromatic amines of the formula $R^1$-$NH_2$ or $R^2$-$NH_2$, where $R^1$ and $R^2$ have the abovementioned meanings, and at least 2 equivalents of a trialkyl orthoformate.

**5.** The use of esters and amides of aminomethylenecyanoacetic acid I as claimed in claims 1 to 3 as stabilizers for organic materials.

**6.** The use of esters or amides of aminomethylenecyanoacetic acid I as claimed in claims 1 to 3 as stabilizers for plastics and surface coatings.

**7.** An organic material which is stabilized against the action of light, oxygen and heat and contains from 0.01 to 5 % by weight, based on the amount of organic material, of one or more eaters or amides of aminomethylenecyanoacetic acid I as claimed in claims 1 to 3.

**8.** A plastic or surface coating which is stabilized against the action of light, oxygen and heat and contains from 0.01 to 5 % by weight, based on the amount of the plastic or surface coating, of one or more eaters or amides of aminomethylenecyanoacetic acid I as claimed in claims 1 to 3.

**9.** A cosmetic preparation containing from 0.1 to 10 % by weight, based on the amount of the cosmetic preparation, of one or more eaters or amides of aminomethylenecyanoacetic acid I as claimed in claims 1 to 3 as sunscreen agent.

**10.** A method for the stabilization of organic materials, which comprises using eaters or amides of aminomethylenecyanoacetic acid I as claimed in claims 1 to 3.

**11.** A method for the stabilization of plastics and surface coatings, which comprises using eaters or amides of aminomethylenecyanoacetic acid I as claimed in claims 1 to 3.

**12.** A method for protecting the human skin from the action of light, which comprises using cosmetic preparations which contain esters or amides of aminomethylenecyanoacetic acid I as claimed in claims 1 to 3 as sunscreen agents.

**Revendications**

**1.** Esters et amides d'acide aminométhylènecyanacétique de formule générale I

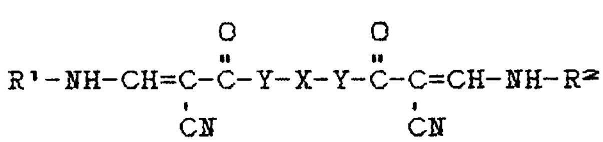

(I)

dans laquelle

$R^1$ et $R^2$ représentent chacun, indépendamment l'un de l'autre, un reste phényle, naphtyle, biphényle ou hétéroaryle à 5 ou 6 chaînons qui renferme un, deux ou trois atomes d'azote ou un atome d'oxygène ou un atome de soufre ou un atome d'azote et un atome d'oxygène ou un atome d'azote et un atome de soufre et qui peut être benzocondensé, ces restes pouvant être substitués par un à trois groupements alkyle en $C_1$-$c_{12}$, groupements alcoxy en $C_1$-$C_{12}$, atomes d'halogène, groupements cyano,

groupements hydroxy ou groupements de formule $COOR^3$, $COR^3$, $CONHR^3$, $OCOR^3$ ou $NHCOR^3$,

$R^3$ étant mis pour un reste alkyle en $C_1$-$C_{12}$, cycloalkyle en $C_5$-$C_8$ ou phényle,

X représente un groupement alkylène en $C_2$-$C_{30}$ qui peut être interrompu par des atomes d'oxygène non voisins, un groupement alcénylène en $C_4$-$C_{12}$ ou alcinylène en $C_4$-$C_{12}$, les liaisons insaturées n'étant pas voisines des atomes d'oxygène de l'ester, un groupement cycloalkylène en $C_5$-$C_8$ ou un groupement phénylène, et

Y est mis pour O ou NH.

**2.** Esters et amides d'acide aminométhylènecyanacétique I selon la revendication 1, dans lesquels $R^1$ et $R^2$ représentent chacun, indépendamment l'un de l'autre, un reste phényle qui peut être substitué par un ou deux groupements alkyle en $C_1$-$C_{12}$, groupements alcoxy en $C_1$-$C_{12}$, atomes de chlore, groupements cyano, groupements hydroxy ou groupements de formule $COOR^3$, $R^3$ ayant les significations données ci-dessus, ou des restes pyridinyle ou 2-pyrimidinyle qui peuvent être substitués par un ou deux groupements alkyle en $C_1$-$C_4$.

**3.** Esters et amides d'acide aminométhylènecyanacétique I selon la revendication 1 ou 2, dans lesquels X représente un groupement alkylène en $C_2$-$C_{12}$,un groupement de formule $-(CH_2CH_2O)_n-CH_2CH_2-$ ou

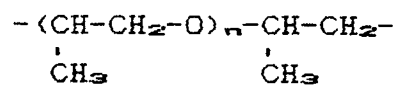

n étant un nombre de 1 à 9, un groupement 1,4-but-2-énylène, un groupement 1,4-but-2-inylène, un groupement cycloalkylène en $C_6$-$C_8$ ou un groupement phénylène.

**4.** Procédé de préparation d'esters et amides d'acide aminométhylènecyanacétique I selon l'une quelconque des revendications 1 à 3, caractérisé en ce que l'on fait réagir des esters ou amides d'acide cyanacétique de formule générale II

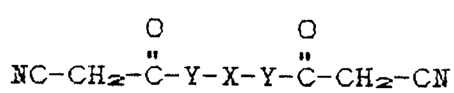

(II)

dans laquelle les variables X et Y ont les significations données ci-dessus, avec 2 équivalents d'amines aromatiques ou hétéroaromatiques de formule générale $R^1$-$NH_2$ ou $R^2$-$NH_2$. où les restes $R^1$ et $R^2$ ont les significations données ci-dessus, et avec au moins 2 équivalents d'un orthoformiate de trialkyle.

**5.** Utilisation d'esters et amides d'acide aminométhylènecyanacétique I selon l'une quelconque des revendications 1 à 3 comme stabilisants pour des matières organiques.

**6.** Utilisation d'esters et amides d'acide aminométhylènecyanacétique I selon l'une quelconque des revendications 1 à 3 comme stabilisants pour des matières plastiques et des laques.

**7.** Matières organiques stabilisées contre l'action de la lumière, de l'oxygène et de la chaleur, contenant de 0,01 à 5% en poids, par rapport à la quantité de la matière organique, d'un ou de plusieurs esters ou amides d'acide aminométhylènecyanacétique I selon l'une quelconque des revendications 1 à 3.

**8.** Matières plastiques et laques stabilisées contre l'action de la lumière, de l'oxygène et de la chaleur, contenant de 0,01 a 5% en poids, par rapport à la quantité de la matière plastique ou de la laque, d'un ou de plusieurs esters ou amides d'acide aminométhylènecyanacétique I selon l'une quelconque des revendications 1 à 3.

**9.** Préparations cosmétiques contenant, en tant qu'agent de protection contre la lumière, de 0,1 à 10% en poids, par rapport à la quantité des préparations cosmétiques, d'un ou de plusieurs esters ou amides d'acide aminométhylènecyanacétique I selon l'une quelconque des revendications 1 à 3.

**10.** Procédé pour la stabilisation de matières organiques, caractérisé en ce qu'on utilise à cette fin des esters ou amides d'acide aminométhylènecyanacétique I selon l'une quelconque des revendications 1 à 3.

**11.** Procédé pour la stabilisation de matières plastiques et de laques, caractérisé en ce qu'on utilise à cette fin des esters ou amides d'acide aminométhylènecyanacétique I selon l'une quelconque des revendications 1 à 3.

**12.** Procédé de protection de la peau humaine contre l'action de la lumière, caractérisé en ce qu'on utilise à cette fin des préparations cosmétiques qui contiennent, en tant qu'agent de protection contre la lumière, des esters ou amides d'acide aminométhylènecyanacétique I selon l'une quelconque des revendications 1 à 3.